# EUROPÄISCHE PATENTSCHRIFT

(11) **EP 2 674 159 B1**
(45) Veröffentlichungstag und Bekanntmachung des Hinweises auf die Patenterteilung: **27.04.2016**
(21) Anmeldenummer: 12004527.3
(22) Anmeldetag: 15.06.2012
(51) Int. Cl.: A61K 31/714, A61K 31/409, A61K 31/4415, A61K 31/525, A61K 36/73, A61K 36/87, A61P 1/16

(54) **Pharmazeutische Zusammensetzung für die Leber-Regeneration**
Pharmaceutical compound for liver regeneration
Composition pharmaceutique pour la régénération du foie

(43) Veröffentlichungstag der Anmeldung: 18.12.2013
(73) Patentinhaber: PHRONTIER S.A.R.L., 76490 Caudebec-en-Caux (FR)
(72) Erfinder: Görne, Martin, 22337 Hamburg (DE)
(74) Vertreter: Diehl & Partner GbR

(56) Entgegenhaltungen:
- EP-A1- 0 595 005
- WO-A1-2004/047566
- DE-A1-102005 009 379
- DE-A1-102007 003 795
- US-A1- 2006 216 361
- US-A1- 2006 233 774

## Beschreibung

Die vorliegende Erfindung bezieht sich auf eine Zusammensetzung zur Steigerung der Leistung der menschlichen Leber, insbesondere einer zirrhotischen Leber.

Es sind bereits Zusammensetzungen bekannt, denen eine heilende Wirkung auf geschädigte Lebern von Säugetieren, insbesondere des Menschen, zugesprochen wurde, z. B. aus den Dokumenten EP 0 101 294 A2 oder WO 03/066058 A1. Die Anmeldungen WO 2004/047566 A1 und US 2006/0233774 A1 offenbaren hepatokurative bzw. hepatoprotektive Zusammensetzungen, die unter anderem die Vitamine A, B₁, B₂, B₆, B₁₂, C, E und B₃ bzw. D sowie Folsäure enthalten. Keine der in diesen Dokumenten vorgeschlagenen Zusammensetzungen hat die in sie gesetzten Hoffnungen tatsächlich erfüllt. Es besteht daher immer noch Bedarf an einer Zusammensetzung, die in der Lage ist, eine klinisch manifeste, z. B. durch eine Noxe verursachte Leber-Funktionsstörung beim Menschen dauerhaft zu lindern. Beim Menschen ist häufig Ethanol die ursächliche Noxe; gleichwohl ist Ethanol-Abusus in Europa (und nicht nur dort) weit verbreitet. Es ist daher Aufgabe der Erfindung, eine pharmazeutische Zusammensetzung zur Steigerung der Leber-Leistung vorzuschlagen.

Diese Aufgabe wird gelöst durch die pharmazeutische Zusammensetzung zur Anwendung in einem Verfahren zur therapeutischen Steigerung der Leber-Leistung gemäß Anspruch 1.

Die Erfindung geht aus von der Beobachtung, dass gewisse Vitamin-Kombinationspräparate eine Senkung von erhöhten Leberenzym-Serumwerten bewirkten. Ausführliche Studien an Patienten mit Leberschädigung und einhergehend erhöhten Blutwerten der einschlägigen Leberenzyme führten zu dem der vorliegenden Erfindung zugrundeliegenden Befund, dass besonders ein relativ scharf umrissener Bereich der mengenmäßigen Verhältnisse der drei Komponenten B6, B12 und Folsäure der Vitaminmischung, und ein gleichermaßen scharf begrenzter Bereich der Gesamt-Tagesdosis, einen deutlichen Effekt auf die Leberenzym-Serumwerte hat. In einem engeren Bereich innerhalb dieses lebertherapeutisch klinisch wirksamen Bereichs wurde auch dann ein günstiger Effekt auf die Leberfunktion festgestellt, wenn die Tagesdosis erheblich, nämlich bis zu einem Faktor 6 erniedrigt war.

Ferner wurde festgestellt, dass in diesem enger begrenzten Bereich auch eine synergistische Wirkung mit der Gabe von solchen Pflanzenextrakten auftrat, die z. B. polyphenolische Antioxidanzien in erheblichem Umfang enthalten. Die Herstellung solcher Extrakte ist in dem europäischen Patent EP 1 190 024 B1 beschrieben. Die Polyphenole weisen mindestens 2, in Ausführungsformen 5 bis 7 Phenylringe und insgesamt mindestens 5, in Ausführungsformen mindestens 12 phenolische Hydroxylgruppen auf.

Die Leber-Leistung wird in diesem Zusammenhang verstanden als Entgiftungsleistung einerseits, messbar als Senkung eines erhöhten Bilirubin-Werts, und als Syntheseleistung andererseits, messbar als Erhöhung eines zuvor erniedrigten Serum-Albuminwerts. Beide hängen ursächlich zusammen.

Im Folgenden werden sowohl absolute Gewichtsangaben für die Tages-Dosen der Vitamin-Komponenten verwendet, als auch relative, die durch Bezug auf RDA-Werte (Recommended Daily Allowance) erhalten wurden. Die hierbei verwendeten RDA-Werte sind 1,3 mg für Vitamin B6, 2,4 µg für Vitamin B12, und 0,4 mg für Vitamin B9 (Folsäure). Insbesondere werden Tagesdosierungen für die Vitamine B6, B9 (Folsäure) und B12 vorgeschlagen, die im Bereich des 1,5- bis 40-fachen der RDA liegen (B9), des 3- bis 380-fachen (B6) bzw. sogar im Bereich des 3- bis 1700-fachen (B12). Dabei liegt in Ausführungsformen ein Gewichtsverhältnis der Vitamine B9 (Folsäure) und B12 im Bereich 0,8:1 oder mehr, insbesondere im Bereich 6:1 bis 10:1. Das Gewichtsverhältnis der Vitamine B6 und B12 liegt im Bereich 40:1 - 1000:1, insbesondere 55:1 - 80:1. Die Tagesdosierungen für die Vitamine B6, B9 (Folsäure) und B12 liegen allgemein bei nicht unter 4 mg (B6), nicht unter 0,6 mg (B9) bzw. nicht unter 0,008 mg (B12). Ferner ist es zweckmäßig, von den Vitaminen nicht über 500 mg (B6) bzw. nicht über 15 mg (B9) bzw. nicht über 4 mg (B12) täglich zu verabreichen. Tages-dosen beziehen sich auf durchschnittsgewichtige Erwachsene.

In einigen Ausführungsformen, in denen der Gewichtsanteil des Vitamin B6 auf unter 95 % oder unter 90 % reduziert, und der Gewichtsanteil des Vitamin B9 auf über 5 % oder über 9 % erhöht ist (wobei der Gewichtsanteil des Vitamin B12 immer untergeordnet ist, nämlich zwischen 0,1 % und 2 % beträgt), ist eine Tagesdosis von unter 60 mg/d der Zusammensetzung bis hinunter zu 8 mg/d hinsichtlich einer Reduktion des Gesamt-Bilirubins im Serum und, damit einhergehend, einer Verbesserung des subjektiv feststellbaren Allgemeinbefindens ausreichend. Die Wirksamkeit kann in diesem Dosisbereich durch die zusätzliche Gabe von mindestens 25 mg/d eines polyphenolischen Antioxidans, z. B. in Form von Traubenkern- oder Aroniaextrakt, signifikant weiter gesteigert werden. Ohne Vitamingabe sind derartige Nahrungszusätze ohne messbaren Einfluss auf die Leberfunktion.

Unter "Vitamin B6" sei im Rahmen dieser Anmeldung eines oder mehrere aus der Gruppe Pyridoxin, Pyridoxal, Pyridoxamin sowie deren Ester, insbesondere Phosphorsäureester verstanden (siehe Anhang B6). Unter "Vitamin B9 (Folsäure)" sei eines oder mehrere aus der Gruppe Folsäure, Folat und deren Metaboliten, nämlich der in Anhang B9 aufgeführten Derivate sowie deren Ester und Amide verstanden. Unter "Vitamin B12" sei eines oder mehrere aus der Gruppe der Cobalamine verstanden, wie sie in Anhang B12 aufgeführt sind. Die Gruppenmitglieder stellen jeweils pharmazeutisch akzeptable Salze oder Derivate der Grundsubstanz dar.

### 1. Beispiel

Gemäß einem ersten Beispiel wurde eine Zusammensetzung mit Vitamin B6, Folsäure und Vitamin B12 im Verhältnis 50:1:1 (jeweils in mg) über einen Zeitraum von 8 Wochen an eine Gruppe von 28 Probanden mit erhöhten Leberfunktionswerten und erhöhtem Gesamtbilirubinwert (Child-Index A bis B) verabreicht: Der Bilirubinwert verringerte sich im Mittel auf 77 % des Ausgangswerts, GOT auf 50 %, GPT auf 57 % und γGT auf 78 %. Nach Absetzen der Therapie stellten sich innerhalb einiger Wochen die Ausgangswerte wieder ein.

### 2. Beispiel

Sodann wurde eine Zusammensetzung mit Vitamin B6, Folsäure und Vitamin B12 im Verhältnis 50:6,5:0,75 (jeweils in mg) über einen Zeitraum von 8 Wochen an eine Untergruppe von 10 dieser Probanden verabreicht: Der Bilirubinwert verringerte sich im Mittel auf 63% des Ausgangswerts, GOT auf 60 %, GPT auf 58 % und γGT auf 76 %. Nach Absetzen der Therapie stellten sich innerhalb einiger Wochen die Ausgangswerte wieder ein.

### 3. Beispiel

Eine Zusammensetzung mit Vitamin B6, Folsäure und Vitamin B12 im Verhältnis 100:1:1 (jeweils in mg) wurde über einen Zeitraum von 8 Wochen an eine Untergruppe von 7 der Probanden verabreicht: Der Bilirubinwert verringerte sich im Mittel auf 92 % des Ausgangswerts, GOT auf 88 %, GPT auf 74 % und γGT auf 89 %. Nach Absetzen der Therapie stellten sich innerhalb einiger Wochen die Ausgangswerte wieder ein.

### 4. Beispiel

Eine Zusammensetzung mit Vitamin B6, Folsäure und Vitamin B12 im Verhältnis 8,0:1,0:0,12 (jeweils in mg) wurde über einen Zeitraum von 8 Wochen an 27 der Probanden verabreicht: Der Bilirubinwert verringerte sich im Mittel auf 84 % des Ausgangswerts, GOT auf 68 %, GPT auf 80 % und γGT auf 79 %. Nach Absetzen der Therapie stellten sich innerhalb einiger Wochen die Ausgangswerte wieder ein.

### 5. Beispiel

Die gleiche Zusammensetzung mit Vitamin B6, Folsäure und Vitamin B12 im Verhältnis 50:1:1 (jeweils in mg) wie im 1. Beispiel wurde über einen Zeitraum von 8 Wochen an eine Untergruppe von 16 der Probanden verabreicht, zusätzlich aber eine Tagesdosis von 210 mg eines Traubenkernextrakts mit 20 % oligozyklischen Polyphenolen: Der Bilirubinwert verringerte sich im Mittel auf 68 % des Ausgangswerts, GOT auf 46,5 %, GPT auf 48 % und γGT auf 63 %. Nach Absetzen der Therapie stellten sich innerhalb einiger Wochen die Ausgangswerte wieder ein.

### 6. Beispiel

Die gleiche Zusammensetzung mit Vitamin B6, Folsäure und Vitamin B12 im Verhältnis 8,0:1; 0:0,12 (jeweils in mg) wie im 4. Beispiel wurde über einen Zeitraum von 8 Wochen an eine Untergruppe von 11 der Probanden verabreicht, zusätzlich eine Tagesdosis von 210 mg des Traubenkernextrakts mit 20 % oligozyklischen Polyphenolen: Der Gesamtbilirubinwert verringerte sich im Mittel auf 73 % des Ausgangswerts, GOT auf 59 %, GPT auf 65 % und γGT auf 66 %. Nach Absetzen der Therapie stellten sich innerhalb einiger Wochen die Ausgangswerte wieder ein.

### Vergleichsbeispiele

Einer Kontrollgruppe von 10 Probanden wurde lediglich der Traubenkernextrakt verabreicht, in derselben Tagesdosis wie oben beschrieben: Innerhalb der achtwöchigen Therapiedauer wurde keine Veränderung der Transaminasenwerte oder des Gesamtbilirubinwertes festgestellt. Es wurde keine weitere Veränderung gemäß 5. und 6. Beispiel festgestellt, wenn die Tagesdosis an oligozyklischen Polyphenolen unter 25 mg lag.

### Ergebnisse

Gemäß 2. und 3. Beispiel sind Zusammensetzungen in Bezug auf eine temporäre Verbesserung der Leberfunktion wirksam, bei denen die Komponenten der Vitamin-B9-Gruppe etwa ebenso konzentriert (nach Gewicht) vorliegen wie die Komponenten der Vitamin-B12-Gruppe, und die Komponenten der Vitamin-B6-Gruppe etwa 40- bis 120fach höher als jene. Insbesondere sind solche Zusammensetzungen wirksam, bei denen das Verhältnis B9/B12 im Bereich zwischen 0,8 und 1,2 liegt.

Gemäß 1. und 4. Beispiel sind Zusammensetzungen in Bezug auf eine temporäre Verbesserung der Leberfunktion in viel geringerer Konzentration wirksam, bei denen die Komponenten der Vitamin-B9-Gruppe etwa 6-10mal so konzentriert (nach Gewicht) vorliegen wie die Komponenten der Vitamin-B12-Gruppe, und die Komponenten der Vitamin-B6-Gruppe etwa 40-bis 60-fach höher als jene der B9-Gruppe. In diesem Bereich ist die Kombination dieser Vitamine also synergistisch.

Gemäß 5. und 6. Beispiel sind Zusammensetzungen in Bezug auf eine temporäre Verbesserung der Leberfunktion in derselben Konzentration wie gemäß 4. Beispiel stärker wirksam, wenn zusätzlich täglich mindestens 25 mg an oligozyklischen Polyphenolen als Antioxidans verabreicht wurden. In diesem Bereich ist die Kombination der Vitamine mit Antioxidantien also auch synergistisch.

Die genannten Zusammensetzungen finden Anwendung zur Leber-Regeneration oder zur Steigerung der Leber-Leistung oder zur Verabreichung an Personen mit eingeschränkter Leber-Funktion aber ohne Hyperhomocysteinämie, zur Verbesserung ihres Allgemeinbefindens. Dazu beträgt die Tagesdosis an damit verabreichtem Vitamin B12 im Bereich 0,1 - 1,2 mg/d; die Tagesdosis an damit verabreichtem Vitamin B9 (Folsäure) im Bereich 1 - 8 mg/d; und die Gesamt-Tagesdosis an damit verabreichter Vitaminzusammensetzung im Bereich 8 - 60 mg/d oder 8 - 20 mg/d (synergistischer Bereich).

Nach Absetzen kehren die gemessenen Serumwerte innerhalb weniger (ca. 5-8) Wochen wieder auf ihre Ausgangswerte zurück, was den Schluss aufdrängt, dass die Verabreichung der beschriebenen Zusammensetzungen keine strukturellen Änderungen in der Leber im Sinne einer Heilung bewirkt, sondern aktiv in günstiger Weise in die Stoffwechselprozesse derart eingreift, dass Kompensationsmechanismen unterstützt werden. Aus dem gleichen Grund ist die Vermeidung von leberschädigenden Noxen wie Ethanol für den Therapieerfolg unabdingbar.

Die Erfindung ist nicht auf die beschriebenen Beispiele beschränkt, sondern ihr Umfang ergibt sich aus den beigefügten Ansprüchen.

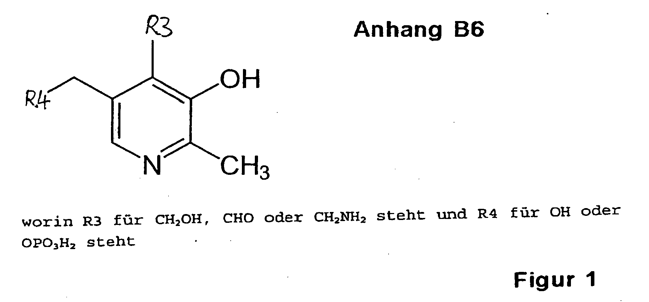

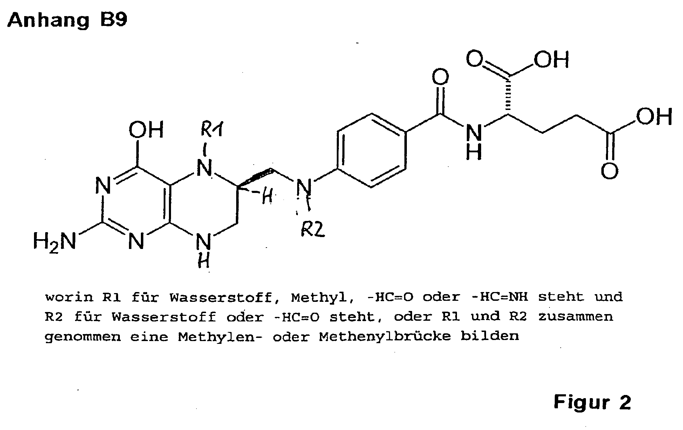

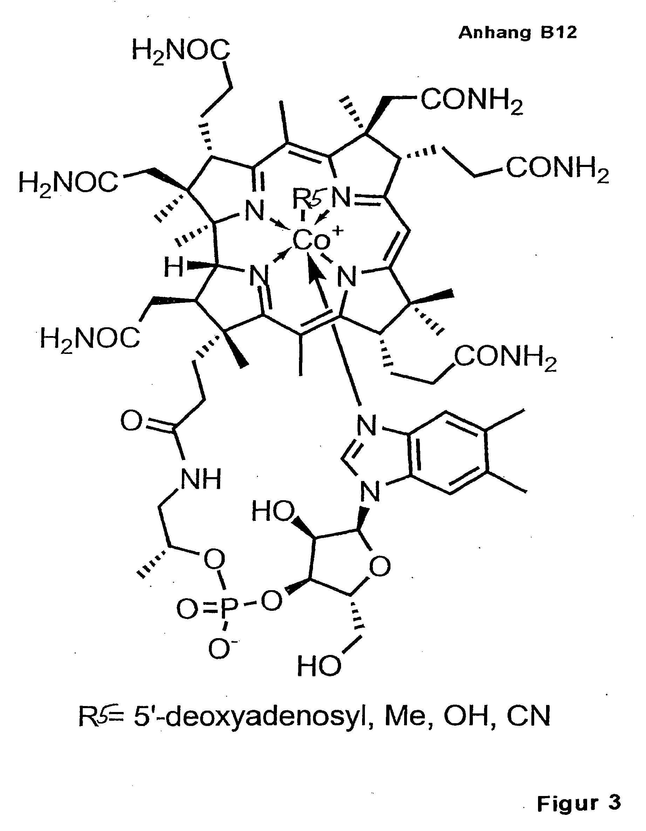

## Patentansprüche

1. Pharmazeutische Zusammensetzung zur Anwendung in einem Verfahren zur therapeutischen Steigerung der Leber-Leistung, die pharmazeutische Zusammensetzung enthaltend die Vitamine B6, B9 (Folsäure) und B 12, **gekennzeichnet durch** die folgenden Tagesdosierungen:
B6 nicht unter 4 mg,
optional nicht mehr als 500 mg;
B9 nicht unter 0,6 mg,
optional nicht über 15 mg;
B 12 nicht unter 0,008 mg,
optional nicht über 4 mg;
wobei ein Gewichtsverhältnis der Vitamine B9 und B12 im Bereich 0,8:1 oder
mehr, und ein Gewichtsverhältnis der Vitamine B6 und B12 im Bereich 40:1 bis 1000:1 liegt.

2. Pharmazeutische Zusammensetzung zur therapeutischen Anwendung nach Anspruch 1, wobei das Verfahren deren orale Verabreichung derart umfasst, dass die Tagesdosis an damit verabreichtem Vitamin B9 (Folsäure) im Bereich 0,8 - 8 mg/d (2-20 RDA) liegt.

3. Pharmazeutische Zusammensetzung zur therapeutischen Anwendung nach Anspruch 1 oder 2, wobei das Verfahren deren orale Verabreichung derart umfasst, dass die Tagesdosis an damit verabreichtem Vitamin B 12 im Bereich 0,008 - 4 mg (3-1700 RDA) oder 0,1-1,2 mg/d (40-500 RDA) oder 0,2-1,2 mg/d (80-500 RDA) liegt.

4. Pharmazeutische Zusammensetzung zur therapeutischen Anwendung nach einem der Ansprüche 1 bis 3, wobei das Verfahren deren orale Verabreichung derart umfasst, dass die Tagesdosis an damit verabreichtem Vitamin B6 im Bereich 4-500 mg (3-380 RDA) oder 6,5 - 117 mg/d (5-90 RDA) liegt.

5. Pharmazeutische Zusammensetzung zur therapeutischen Anwendung nach einem der Ansprüche 1 bis 4, wobei die pharmazeutische Zusammensetzung die Vitamine B6 und B9 (Folsäure) im Gewichtsverhältnis von 2:1 bis 12:1 oder optional mehr als 6:1 und optional weniger als 9:1 enthält.

6. Pharmazeutische Zusammensetzung zur therapeutischen Anwendung nach Anspruch 5, enthaltend die Vitamine B9 (Folsäure) und B 12 im Gewichtsverhältnis 6:1 bis 10:1.

7. Pharmazeutische Zusammensetzung zur therapeutischen Anwendung nach einem der Ansprüche 1 bis 6, enthaltend die Vitamine B6 und B12 im Gewichtsverhältnis 55:1 bis 80:1.

8. Pharmazeutische Zusammensetzung zur therapeutischen Anwendung nach Anspruch 7, enthaltend die Vitamine B9 (Folsäure) und B 12 im Gewichtsverhältnis 7:1 - 9:1.

9. Pharmazeutische Zusammensetzung zur therapeutischen Anwendung nach Anspruch 8, wobei das Gewichtsverhältnis B6:B9 (Folsäure) in Bereich 6:1-9:1 liegt.

10. Pharmazeutische Zusammensetzung zur therapeutischen Anwendung nach Anspruch 8 oder 9, wobei die Gesamt-Tagesdosis an verabreichter VitaminZusammensetzung im Bereich 8 - 60 mg/d oder 8 - 20 mg/d liegt.

11. Pharmazeutische Zusammensetzung zur therapeutischen Anwendung nach einem der Ansprüche 5 bis 10, wobei das Verfahren ferner die Verabreichung einer Tagesdosis von mindestens 25 mg/d Polyphenol-Antioxidans umfasst.

12. Pharmazeutische Zusammensetzung zur therapeutischen Anwendung nach Anspruch 11, wobei Traubenkern- oder Aroniaextrakt als Antioxidans-Quelle verabreicht wird.

13. Pharmazeutische Zusammensetzung zur therapeutischen Anwendung nach einem der Ansprüche 7 bis 12, wobei das Verfahren deren orale Verabreichung derart umfasst, dass die Tagesdosis an damit verabreichtem Vitamin B12 im Bereich 0,01 - 0,6 mg/d (4-250 RDA) liegt.

14. Pharmazeutische Zusammensetzung zur therapeutischen Anwendung nach einem der Ansprüche 1 bis 4, wobei die pharmazeutische Zusammensetzung die Vitamine B9 (Folsäure) und B12 im Gewichtsverhältnis 4:5 bis 5:4 enthält und das Verfahren die orale Verabreichung der pharmazeutische Zusammensetzung derart umfasst, dass die Tagesdosis an damit verabreichtem Vitamin B6 im Bereich 40 - 120 mg/d (30-90 RDA) liegt.

15. Pharmazeutische Zusammensetzung zur therapeutischen Anwendung nach einem der vorstehenden Ansprüche, zur Verabreichung an Personen mit eingeschränkter Leber-Funktion aber ohne Hyperhomocysteinämie.

## Claims

1. A pharmaceutical composition for use in a method of therapeutic enhancement of liver performance, the pharmaceutical composition comprising the vitamins B6, B9 (folic acid) and B12, **characterized by** the following daily doses:
B6 not below 4 mg, optionally not more than 500 mg;
B9 not below 0.6 mg, optionally not above 15 mg;
B12 not below 0,008 mg, optionally not above 4 mg;
wherein a weight ratio of the vitamins B9 and B12 is in a range 0,8:1 or more, and a weight ratio of the vitamins B6 and B12 is in a range 40:1 to 1000:1.

2. The pharmaceutical composition for therapeutic use according to claim 1, wherein the method comprises orally administering same such that the daily dose of vitamin B9 (folic acid) so administered is in the range 0.8 - 8 mg/d (2-20 RDA).

3. The pharmaceutical composition for therapeutic use according to claim 1 or 2, wherein the method comprises orally administering same such that the daily dose of vitamin B12 so administered is in the range 0.008 - 4 mg (3-1700 RDA) or 0.1-1.2 mg/d (40-500 RDA) or 0.2-1.2 mg/d (80-500 RDA).

4. The pharmaceutical composition for therapeutic use according to one of claims 1 to 3, wherein the method comprises orally administering same such that the daily dose of B6 so administered is in the range 4-500 mg (3-380 RDA) or 6.5 - 117 mg/d (5-90 RDA).

5. The pharmaceutical composition for therapeutic use according to one of claims 1 to 4, wherein the pharmaceutical composition comprises the vitamins B6 and B9 (folic acid) in a weight ratio of from 2:1 to 12:1 or optionally more than 6:1 and optionally less than 9:1.

6. The pharmaceutical composition for therapeutic use according to claim 5, comprising the vitamins B9 (folic acid) and B12 in a weight ratio of 6:1 to 10:1.

7. The pharmaceutical composition for therapeutic use according to one of claims 1 to 6, comprising the vitamins B6 and B12 in a weight ratio of 55:1 to 80:1.

8. The pharmaceutical composition for therapeutic use according to claim 7, comprising the vitamins B9 (folic acid) and B 12 in a weight ratio of 7:1 - 9:1.

9. The pharmaceutical composition for therapeutic use according to claim 8, wherein the weight ratio B6:B9 (folic acid) is in the range 6:1-9:1.

10. The pharmaceutical composition for therapeutic use according to claim 8 or 9, wherein the total daily dose of administered vitamin composition is in the range 8 - 60 mg/d or 8 - 20 mg/d.

11. The pharmaceutical composition for therapeutic use according to one of claims 5 to 10, wherein the method further comprises the administration of a daily dose of at least 25 mg/d polyphenolic antioxidant.

12. The pharmaceutical composition for therapeutic use according to claim 11, wherein grapeseed or aronia extract is administered as the source of the antioxidant.

13. The pharmaceutical composition for therapeutic use according to one of claims 7 to 12, wherein the method comprises its oral administration such that the daily dose of vitamin B12 so administered is in the range 0.01 - 0.6 mg/d (4-250 RDA).

14. The pharmaceutical composition for therapeutic use according to one of claims 1 to 4, wherein the pharmaceutical composition contains the vitamins B9 (folic acid) and B12 in a weight range of 4:5 to 5:4 and the method comprises the oral administration of the pharmaceutical composition such that the daily dose of vitamin B6 so administered is in the range 40 - 120 mg/d (30-90 RDA).

15. The pharmaceutical composition for therapeutic use according to one of the preceding claims, for administration to persons suffering limited liver function but no hyperhomocysteinaemia.

## Revendications

1. Composition pharmaceutique pour une application dans un procédé pour l'amélioration thérapeutique de l'action hépatique, la composition pharmaceutique contenant les vitamines B6, B9 (acide folique) et B12,
**caractérisée par** les dosages journaliers suivants :
pas moins que 4 mg de vitamine B6, optionnellement pas plus que 500 mg ;
pas moins que 0,6 mg de vitamine B9, optionnellement pas supérieur à 15 mg ;
pas moins que 0,008 mg de vitamine B12, optionnellement pas supérieur à 4 mg ;
un rapport en poids entre les vitamines B9 et B12 étant égal à 0,8:1 ou plus, et un rapport en poids entre les vitamines B6 et B12 se situant dans la plage allant de 40:1 à 1000:1.

2. Composition pharmaceutique d'application thérapeutique selon la revendication 1, le procédé incluant son administration orale de telle manière que la dose journalière en vitamine B9 (acide folique) ainsi administrée se situe dans la plage de 0,8 - 8 mg/j (2 - 20 RDA).

3. Composition pharmaceutique d'application thérapeutique selon la revendication 1 ou 2, le procédé incluant son administration orale de telle manière que la dose journalière en vitamine B12 ainsi administrée se situe dans la plage de 0,008 - 4 mg (3 - 1700 RDA) ou de 0,1 - 1,2 mg/j (40 - 500 RDA) ou de 0,2-1,2 mg/j (80 - 500 RDA).

4. Composition pharmaceutique d'application thérapeutique selon l'une quelconque des revendications 1 à 3, le procédé incluant son administration orale de telle manière que la dose journalière en vitamine B6 ainsi administrée se situe dans la plage de 4 - 500 mg (3 - 380 RDA) ou de 6,5 - 117 mg/j (5 - 90 RDA).

5. Composition pharmaceutique d'application thérapeutique selon l'une quelconque des revendications 1 à 4, la composition pharmaceutique contenant les vitamines B6 et B9 (acide folique) selon un rapport en poids allant de 2:1 à 12:1 ou optionnellement supérieur à 6:1, et optionnellement inférieur à 9:1.

6. Composition pharmaceutique d'application thérapeutique selon la revendication 5, contenant les vitamines B9 (acide folique) et B12 selon uh rapport en poids de 6:1 à 10:1.

7. Composition pharmaceutique d'application thérapeutique selon l'une quelconque des revendications 1 à 6, contenant les vitamines B6 et B12 selon un rapport en poids de 55:1 à 80:1.

8. Composition pharmaceutique d'application thérapeutique selon la revendication 7, contenant les vitamines B9 (acide folique) et B12 selon un rapport en poids de 7:1 à 9:1.

9. Composition pharmaceutique d'application thérapeutique selon la revendication 8, le rapport en poids B6:B9 (acide folique) se situant dans la plage 6:1 - 9:1.

10. Composition pharmaceutique d'application thérapeutique selon la revendication 8 ou 9, la dose journalière totale en la composition de vitamines administrée se situant dans la plage de 8 - 60 mg/j ou de 8 - 20 mg/j.

11. Composition pharmaceutique d'application thérapeutique selon l'une quelconque des revendications 5 à 10, le procédé incluant en outre l'administration d'une dose journalière d'au moins 25 mg/j d'un antioxydant à base de polyphénol.

12. Composition pharmaceutique d'application thérapeutique selon la revendication 11, un extrait de pépins de raisin ou un extrait d'aronia étant administré en tant que source d'antioxydant.

13. Composition pharmaceutique d'application thérapeutique selon l'une quelconque des revendications 7 à 12, le procédé incluant en outre son administration orale de telle manière que la dose journalière en vitamine B12 ainsi administrée se situe dans la plage de 0,01 - 0,6 mg/j (4 - 250 RDA).

14. Composition pharmaceutique d'application thérapeutique selon l'une quelconque des revendications 1 à 4, la composition pharmaceutique contenant les vitamines B9 (acide folique) et B12 selon le rapport en poids de 4:5 à 5:4, et le procédé incluant l'administration orale de la composition pharmaceutique de telle manière que la dose journalière en vitamine B6 ainsi administrée se situe dans la plage de 40 - 120 mg/j (30 - 90 RDA).

15. Composition pharmaceutique d'application thérapeutique selon l'une quelconque des revendications précédentes, destinée à être administrée à des patients atteints de troubles de la fonction hépatique mais sans hyperhomocystéinémie.
